# EUROPEAN PATENT SPECIFICATION

(11) **EP 3 820 362 B1**
(45) Date of publication and mention of the grant of the patent: **26.10.2022**
(21) Application number: 19753235.1
(22) Date of filing: 03.07.2019
(51) Int. Cl.: A61B 5/00, A61B 5/291, A61B 5/377

(54) **RECORDING ELECTRODE FOR ELECTROPHYSIOLOGICAL SIGNAL MEASUREMENT**
AUFZEICHNUNGSELEKTRODE ZUR MESSUNG VON ELEKTROPHYSIOLOGISCHEN SIGNALEN
ÉLECTRODE D'ENREGISTREMENT POUR MESURE DE SIGNAL ÉLECTROPHYSIOLOGIQUE

(30) Priority: 09.07.2018 CZ 20180339
(43) Date of publication of application: 19.05.2021
(73) Proprietor: DEYMED Diagnostic s.r.o., 549 31 Hronov (CZ)
(72) Inventor: MORAVEC, Miroslav, 549 31 Hronov (CZ)
(74) Representative: Pavlica, Tomas
(86) International application number: PCT/CZ2019/000030
(87) International publication number: WO 2020/011290

(56) References cited:
- US-A1- 2017 071 490
- US-B1- 9 763 590

## Description

### Field of invention

The present invention relates to a recording electrode suitable for measuring electrophysiological signal in the application of magnetic stimulation, for example when measuring the electrical activity of the brain registered by surface electrodes on the head surface (EEG). The recording electrode according to this invention is particularly preferably suitable for signal measuring with transcranial magnetic stimulation (TMS), since this recording electrode is particularly appropriate for measuring the electrophysiological signal in locations that are in close proximity to the place of transcranial magnetic stimulation.

### State of art

There are currently a number of recording electrodes used for electrophysiological signal measurement. For the purpose of this invention, the recording electrode means an electrode designed to connect the amplifier input circuits to the electrical potential of biological tissue.

Transcranial magnetic stimulation, TMS, which is a stimulation through the skull or spine by the action of induced electrical voltage that is induced by rapid time change of magnetic induction, is currently becoming increasingly important. The induced electrical voltage in biological tissue during TMS is primarily used to stimulate the brain or spinal cord because their direct non-invasive electrical stimulation is complicated and very painful. Transcranial magnetic stimulation is performed by a short magnetic field pulse of the intensity of the order of tesla and of the duration of the order of several hundred microseconds, while the location of registration of the evoked electrophysiological signals in the biological tissue is often in the immediate vicinity of the place of stimulation. The measurement of electrophysiological signals during transcranial magnetic stimulation is typically accompanied by 3 common types of technical artifacts related directly to the recording electrodes, the technical artifact being the portion of the electrophysiological signal registered during such stimulation, which does not have a physiological origin and thus distorts the resulting measurement. For the purposes of this application, electrophysiological signal means a signal showing the electrical activity of cells. The evoked response for the purposes of this application means the electrophysiological activity of nerve cells induced by the action of the stimulation signal.

The common types of technical artifacts, hereinafter referred to only as artifacts, accompanying the measurement of electrophysiological signals during TMS are:
Type A) The magnetically induced artifact, which in its shape accurately replicates the course of the magnetic field change over time. It always has a zero DC component of induced voltage. This technical artifact has such a high component of electric and magnetic fields that it is practically impossible to suppress it to the extent that it does not affect the electrophysiological signal. However, it occurs a very short time. When acquiring continuous recording, it can be masked relatively easily and when measuring evoked, i.e., induced responses, it does not interfere with this response when the signal is processed appropriately, as it does not coincide with it.
Type B) Artifact caused by the heating of the recording electrodes, which is induced by the heat loss generated by eddy currents in large undivided electrically conductive electrode surfaces. This artifact can cause burns to the skin or other biological tissue. In the electrophysiological signal, it exhibits a relatively low amplitude, but with a very long decay time (hundreds of ms to seconds). The shape of this artifact replicates the temperature changes in the electrode or in its vicinity. This artifact is currently solved by dividing the area of the recording electrode so that it does not contain a large continuously electrically well-conductive closed loop.
Type C) A relaxing artifact, which is caused by the balancing of electrochemical changes in biological tissue or in the electro-conductive gel after the stimulus has subsided. These changes occurred during a short TMS stimulus due to the passage of electric current, which was generated by induced electrical voltage. Their return to their original equilibrium values typically lasts tens to hundreds of ms, thus interfering with the evoked responses or, as appropriate, the continuous recording. In the electrophysiological signal, it manifests itself in an exponentially decreasing shape with beginning at the moment of stimulation. The time constant of its fading varies in the order of tens of ms. This artifact is very difficult to get rid of, it tends to be very variable and reaches high amplitudes. In literature it is called "decay", mostly without explaining the causes of its origin. Currently, it is eliminated by software mainly using the Independent Component Analysis (ICA) algorithm.

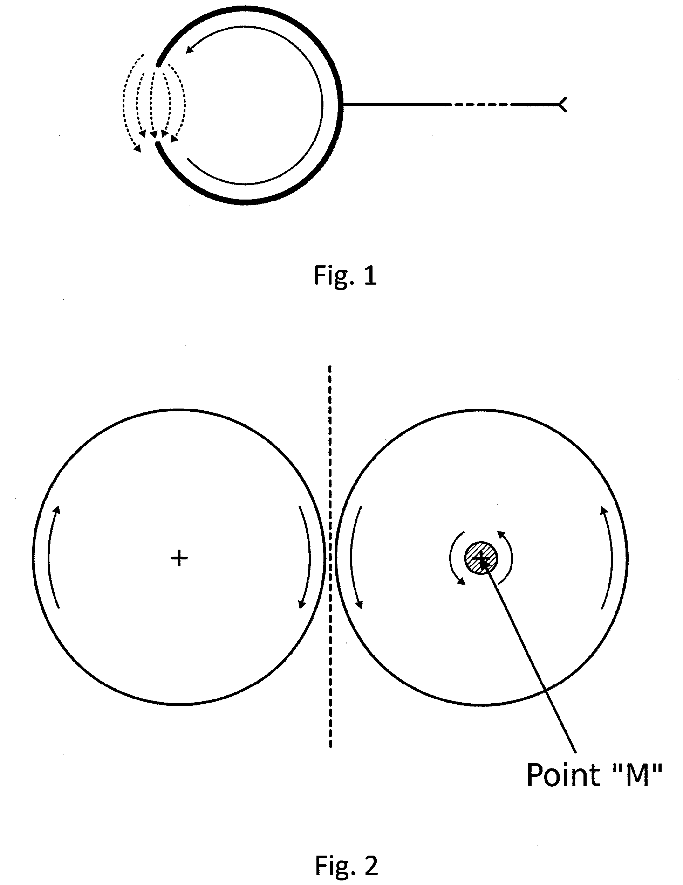

The prior art recording electrodes suitable for TMS can perfectly eliminate the artifacts caused by heating due to the action of eddy currents (type B artifact), but do not address the relaxation artifacts (type C artifact) in any way. On the contrary, they can help with their shape the creation of relaxation artifacts (type C artifacts) by concentrating the induced voltage into a substantially smaller portion of the biological tissue or the electro-conductive gel, see e.g. Fig. 1.

Prior art electrodes are used predominantly in combination with 8-figure stimulation coils (see Fig. 2), where the axis of these coils has approximately laminar distribution of magnetic field lines. There is almost no relaxation artifact (type C artifact) occurring in this axis. The relaxation artifact (type C artifact) begins to appear at other points outside the axis of the 8-figure stimulus coil and it becomes a crucial problem especially at points close to the point of magnetic stimulation M.

Fig. 1 is an example of the solution of prior art recording electrodes suitable for TMS, which prevent the formation of type B artifacts by preventing the generation of eddy currents in the actual recording electrode. In this case, the shape of the recording electrode prevents the formation of a long and closed electrically conductive loop. Although this and other types of recording electrode shapes are divided into several parts, they together form a continuously electrically conductive whole. The disadvantage of these known recording electrodes is that they help to create type C artifacts as a result of facilitating the formation of currents under the electrode, while at the point of interruption of the enclosed electrically conductive loop, the electrical stress of the biological tissue or electro-conductive gel is concentrated precisely in this point and due to the non-linear electrical properties of the biological tissue or the electro-conductive gel, the effect of the formation of currents outside the electrode is multiplied. The arrows from the solid lines show the induction of electrical voltage to the metal part of the electrode. The arrows from the dashed lines then indicate the location of the formation of currents below the electrode. These currents then cause disturbance of the registered signal resulting from the generated artifacts described above, whose fading has the form of an exponential curve with a duration in the order of tens to hundreds of ms.

Another variant of the TMS compatible recording electrodes is the use of a material with high resistivity (e.g., graphite or a very thin metal layer applied to an electrically non-conductive substrate). The disadvantage of these TMS compatible electrodes is the need to make a compromise when selecting the resistivity value so that, on the one hand, a high electrical resistance value is achieved in the direction of the electrode plane and, on the other hand, an acceptable size of the resulting virtual additional transient resistance is achieved. This is due to the isotropic conductivity of almost every commonly used electrode material. The compromise is therefore very imperfect since either the full suppression of the type C artifacts is not achieved or the additional impedance size is too high. There is therefore a need for a recording electrode, which would minimise the interfering signals/artifacts both immediately after stimulation (units of ms) and in the long term (hundreds of ms to seconds).

The above-mentioned disadvantages are eliminated by the recording electrode according to the present invention, which, in addition to eliminating the formation of the type B artifacts by eddy currents also solves the suppression of the type C artifacts.

US 2017/071490 A1 discloses electrodes for measuring of a neural response to a stimulus.

### Subject of the invention

According to this invention, a recording electrode is presented for measuring the electrophysiological signal that substantially prevents the flow of currents due to the concentration of electrical voltage on a small portion of the substance below the recording electrode, that is, on the electro-conductive gel or the biological tissue. By its presence, the recording electrode according to the invention completely eliminates or at least substantially reduces the influence of the registered electrophysiological potential on the surface of the biological tissue with which the recording electrode is in direct contact or with which it is in contact through the electro-conductive gel.

The recording electrode according to the invention can advantageously be used for magnetic resonance examination, where interfering voltages are induced into the recording electrodes or the substance that is in contact with them in a similar manner. The recording electrode according to the present invention can also be used to scan electrophysiological potentials during magnetic stimulation of peripheral nerves or muscles and in other applications. According to the inventor's idea accompanying the summary of this recording electrode, the induced currents do not necessarily have the character of eddy currents only; on the contrary, due to the shapes of the prior art types of electrodes they are often accompanied by other currents that are approximately laminar and lead from one part of the recording electrode to the other, but they pass through the substance with which the electrode is in direct contact. Since this substance is essentially a full or partial electrolyte, whether applied (e.g., electro-conductive gel) or biological, the passage of these currents will result in a violation of its electrochemical equilibrium and thus in its "charging". After the stimulation pulse is terminated, the levelling of these charges occurs, but this gradual levelling creates an exponentially fading type B artifact in the measured electrophysiological signal.

To reliably acquire electrophysiological potentials from biological tissue, it is necessary that the recording electrode according to the invention be attached to the biological tissue through a certain contact surface, determined according to predefined standards. The size of the contact surface is preferably chosen as a compromise between the possibility of achieving the lowest possible size of transient impedance between the recording electrode and the biological tissue, and the possibility of achieving the best spatial resolution.

The subject matter of the recording electrode according to the invention consists in dividing the total contact surface of the recording electrode by which the recording electrode is in contact with the substance located below the recording electrode into at least two electrically separated contact areas and leading each of these separate contact areas through a separate resistor to the output of the recording electrode, wherein the size of the electrical resistance of each of these resistors is at least 100 Ω, while the resulting resistance value of all resistors connecting the individual separate contact areas with the output of the recording electrode, measured between the substance with which the contact area of the recording electrode is in contact and the electrode outlet, is at least 50 Ω, which corresponds to at least half the impedance size of the biological tissue in magnetic stimulation. The biological tissue impedance without stimulation for conventional recording electrodes typically ranges around 1-10 kΩ, depending heavily on the condition of the biological tissue, its preparation, the use or non-use of the electro-conductive gel, as well as on the area, shape and design of the electrode. For the purposes of this application, the substance with which the contact area of the recording electrode is in contact is intended to mean a biological tissue or an electro-conductive gel, which ensures reliable contact of the contact area with the biological tissue. However, in the case of magnetic stimulation of the biological tissue, the induced current causes an avalanche reduction in the biological tissue impedance. According to the present invention, it has been found that the size of the biological tissue impedance during magnetic stimulation is approx. 100 Ω. Based on this finding, the resistance value of one resistor of at least approximately 100 Ω was chosen for the recording electrode according to this invention with at least two electrically separated contact areas. If this value is at least 100 Ω, then the size of the electrical resistance of each resistor is at least roughly the size of the biological tissue impedance between the individual points of contact of the individual separate areas of the contact surface with the biological tissue under magnetic stimulation. The resulting resistance value between the two sensing surfaces then corresponds to at least twice the skin impedance, which is essentially the limit value from which the effect of the type C artifact suppression becomes noticeably apparent. However, the resistance size of the individual resistors further depends on the number of separate contact areas, where, due to the essentially parallel connection of all resistances to each other, it is necessary to ensure that the resulting resistance of all resistors is at least 50 Ω. According to the preferred embodiment, the resistance size of the individual resistors can be up to 10,000 Ω.

Each separate contact area of the recording electrode according to the invention is separately led through resistors of such resistance size so that the resulting value of their resistance (measured between the biological tissue and the recording electrode output) is at least 100 Ω in order that the mutual impedance between any two contact areas is as high as possible while retaining the resultant impedance at the output of the recording electrode as low as possible, thus avoiding any influence on the measurement. Depending on the number of separate contact areas connected through the individual resistors with the output from the recording electrode, the resistance size of the individual resistors through which the individual separate contact areas are connected with the output from the recording electrode according to the present invention should essentially constitute a compromise when it should be high enough to prevent the formation of currents below the recording electrode and at the same time low enough not to increase the effective transient impedance of the skin - output from the recording electrode. The higher number of electrically separated acquiring surfaces will thus allow to reduce the resulting impedance of the acquiring surfaces, as measured between the substance with which the contact area is in touch and the output from the recording electrode, while maintaining a relatively high impedance between any two acquiring surfaces of the recording electrode.

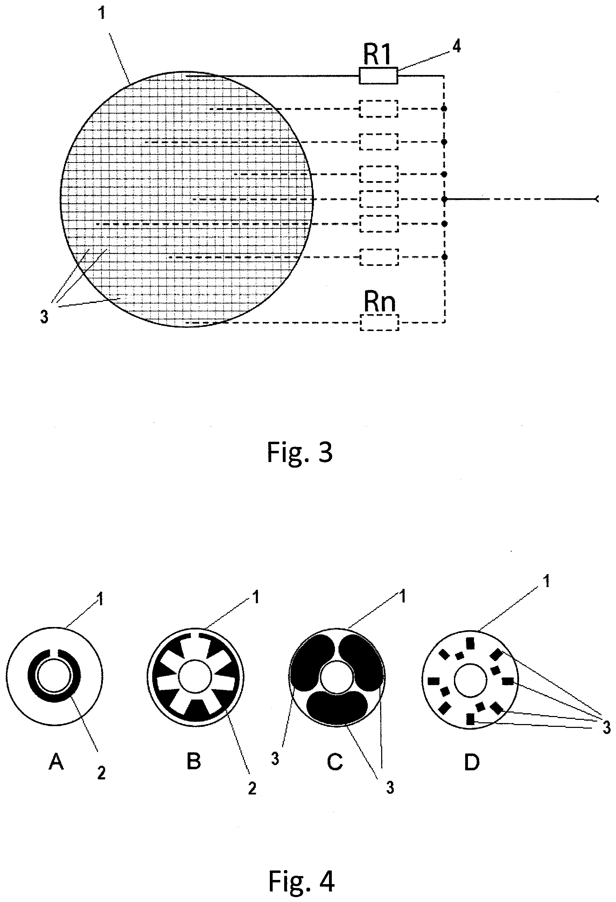

More preferably, the recording electrode for electrophysiological signal measurement according to the present invention is created with the division of the recording electrode surface into 3 to 12 separate contact areas. These separate contact areas are each connected to the output of the recording electrode through a separate resistor of the appropriate resistance value, preferably at least 200 Ω and even more preferably at least 1 kΩ. Therefore, the resulting resistance value between any two separate contact areas of the recording electrode is at least approx. 100 Ω, more preferably in the order of hundreds of Ω, i.e., for example, in 200 Ω resistors it is 400 Ω, and particularly preferably in the order of units of kΩ, e.g. in 1 kΩ resistors this value is 2 kΩ. According to the theory inherent in this invention, although it appears particularly advantageous, when the recording electrode is made up of as many electrically separated small areas as possible, see, e.g., Fig. 3, this number is in practice limited by technological possibilities and, moreover, from a certain number of separate areas, the suppression of the type C artifact increases only slightly. It is for this reason that the recording electrode according to the invention comprises in its most preferred embodiment 12 electrically separated small areas which are connected to the electrode output through resistors having a total resistance size of at least 50 Ω, more preferably at least 100 Ω, while the individual resistances are preferably at least 1 kΩ and most preferably up to 10 kΩ. If there is a lower number of the electrically separated contact areas of the recording electrode, the transient impedance between the electrode and the biological tissue is uselessly increased. This is unnecessary, since multiple separate contact areas can be easily placed on the electrode. On the other hand, with a higher number of contact areas than 12, it could be problematic to arrange the areas in space reasonably and bring the resistors to them. This, however, does not exclude a larger number of separate contact areas if technological possibilities allow it or if it is needed for some reason. When the recording electrode is divided into 12 electrically separated contact areas, it has been found to be particularly advantageous if the size of the individual resistors by which the separate areas are connected to the output of the recording electrode is approx. 2 kΩ, due to which the total output impedance of the recording electrode will increase by only approx. 200 Ω, which is an acceptable value in respect of the electrical resistance of the biological tissue, which does not distort the final electrophysiological signal. The value of the resistance between any two separate contact areas of the recording electrode is 4 kΩ, which ensures their reliable electrical separation in terms of the formation of the type C artifact. This high value is a very good compromise ensuring a minimal increase in the electrode impedance, while very well eliminating the formation of the type B artifacts.

As mentioned above, the size of the individual separate areas of the recording electrode helps to prevent the formation of artifacts caused by the thermal heating of the electrode due to eddy currents. The values of the size of separate areas up to approx. 15 mm² are preferable in this aspect. Particularly preferably, the recording electrode contains separate areas with a size of approx. 1.5 mm x 1.5 mm, i.e. an area of approx. 2 mm².

Thus, according to its particularly preferable embodiment, the recording electrode according to the invention is divided into 12 separate electrically separated contact areas with a size of approx. 1.5 x 1.5 mm², which are connected to the output from the recording electrode always through the respective resistors of approx. 2 kΩ.

According to this invention, it is also envisaged to use an anisotropically conductive material of the recording electrode, which would allow low resistance in the direction of the recording electrode surface relative to the electrode outlet, but at the same time would ensure sufficient separation of the individual recording electrode surfaces relative to each other.

The recording electrode according to this invention is particularly advantageous for TMS applications because it minimises eddy current formation in the electrode itself, but it in particular prevents or at least minimises the formation of additional currents in the substance located below this electrode, which is in direct contact with the recording electrode, where the said substance is the biological tissue to which the recording electrode is attached or, as appropriate, the electro-conductive gel used to provide a reliable electrical connection of the recording electrode with the biological tissue.

### Description of drawings

The invention will be more clearly understandable from the examples of the recording electrode embodiment and from the attached drawings, in which:
Fig. 1 - Represents an example of a prior art recording electrode,
Fig. 2 - Marking of magnetic field lines when using octal coil,
Fig. 3 - Represents an ideal embodiment of a TMS compatible recording electrode according to the invention,
Fig. 4 - Represents the embodiment of four compared TMS compatible recording electrodes,
Fig. 5 - Represents a prior art electrode B from Fig. 4 and a similar electrode adapted according to the present invention,
Fig. 6 - Signal from electrode A from Fig. 4,
Fig. 7 - Signal from electrode B from Fig. 4,
Fig. 8 - Signal from electrode C from Fig. 4,
Fig.. 9 - Signal from electrode E from Fig. 5,
Fig. 10 - Signal from electrode F from Fig. 5,

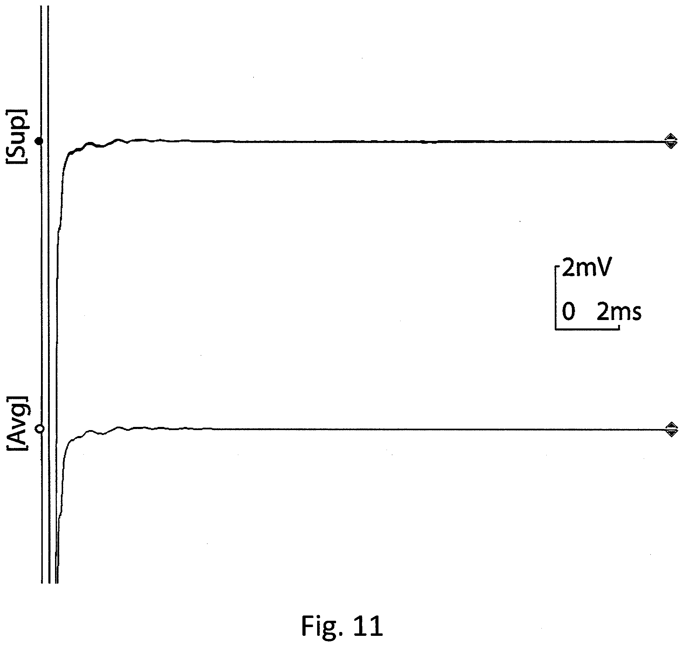

Fig. 11 - Signal from electrode D from Fig. 4 with resistances of 0.1 kΩ,
Fig. 12 - Signal from electrode D from Fig. 4 with resistances of 1.5 kΩ,
Fig. 13 - 100x zoomed signal Fig. 12,
Fig. 14 - Signal from electrode D from Fig. 4 with resistances of 10 kΩ,
Fig. 15 - 100x zoomed signal Fig. 14.

### Examples of embodiment

The invention will be illustrated on the following examples of embodiment. These examples are for illustrative purposes only and are used to understand the essence of the invention. They should in no way be understood as the only possible examples of embodiment, which would limit the invention to the embodiment examples depicted and described herein.

In the following embodiment examples, the prior art recording electrodes and various embodiments of the recording electrode according to the present invention will be described for understanding the advantages of the recording electrode according to the invention.

Fig. 1 serves to understand the essence of the invention and shows an example of prior art recording electrode 1, where the thick line shows the course of induced currents in the electrode material, while the dashed lines show the course of induced currents in the substance below the electrode, i.e. in the skin or in the gel. The figure also shows the course of induced currents in the prior art recording electrode 1 of type A from Fig. 4.

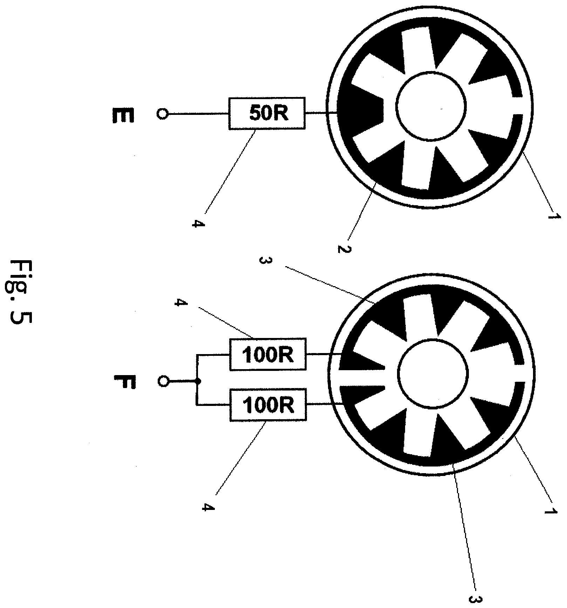

Table 1 gives an overview of all the recording electrodes 1 from Fig. 4 and 5, which are compared in the embodiment examples. Table 1 also shows which figure shows the resulting curves measured at the output of each particular recording electrode 1.

**Table 1**

| Electrode identification | Electrode description | Number of (separated) areas | Resistors used | Size of the resulting resistance | Signal flow shown on |
|---|---|---|---|---|---|
| A in Fig. 4 | Cut ring | 1 | - | 0 Ω | Fig. 6 |
| B in Fig. 4 | Cut ring with surfaces | 1 | - | 0 Ω | Fig. 7 |
| C in Fig. 4 | 3x larger surface | 3 | 10 kΩ | 3.333 kΩ | Fig. 8 |
| D in Fig. 4 | 12x smaller surface | 12 | 0.1 kΩ | 0.00833 kΩ | Fig. 11 |
| D in Fig. 4 | 12x smaller surface | 12 | 1.5 kΩ | 0.125 kΩ | Fig. 12 |
| | | | | | Fig. 13 |
| D in Fig. 4 | 12x smaller surface | 12 | 10 kΩ | 0.833 kΩ | Fig. 14 |
| | | | | | Fig. 15 |
| E in Fig. 5 | Undivided electrode | 1 | 50 Ω | 50 Ω | Fig. 9 |
| F in Fig. 5 | Divided electrode | 2 | 100 Ω | 50 Ω | Fig. 10 |

Stimulation was performed by a biphasic magnetic pulse of approx. 2 T amplitude and a duration of approx. 300 µs. The vector of time-varying magnetic induction showed the highest possible vortex character at the point of measurement. These conditions appear to be the most demanding within the commonly used TMS stimulation parameters to suppress the type B artifact.

Several measurements were made to determine the highest acceptable continuous area and the size of the recording electrode resistors. The results were also compared with electrode types that do not use separating resistors.

Fig. 3 shows an ideal recording electrode 1 divided into n separate contact areas 3, where each separate contact surface 3 is led through its resistor 4 to the output from the recording electrode 1. To simplify the understanding of this recording electrode 1, only some resistors 4 are shown through which the separated contact areas 3 are led out.

### Example 1

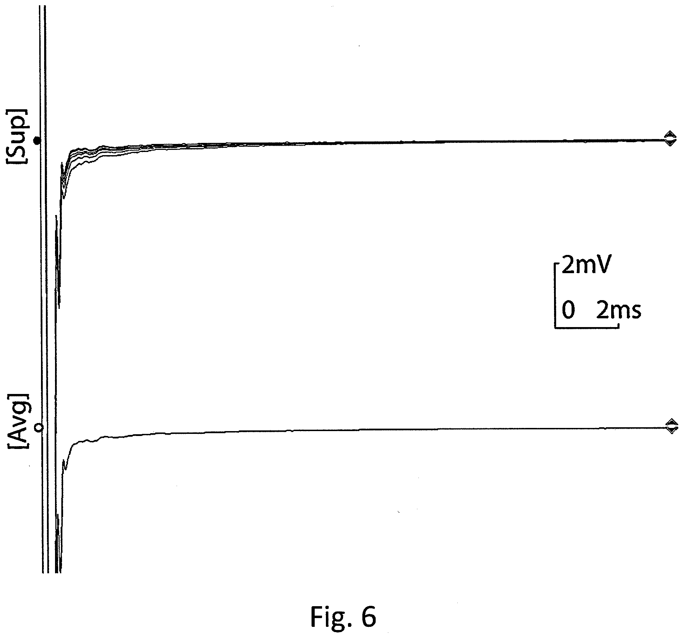

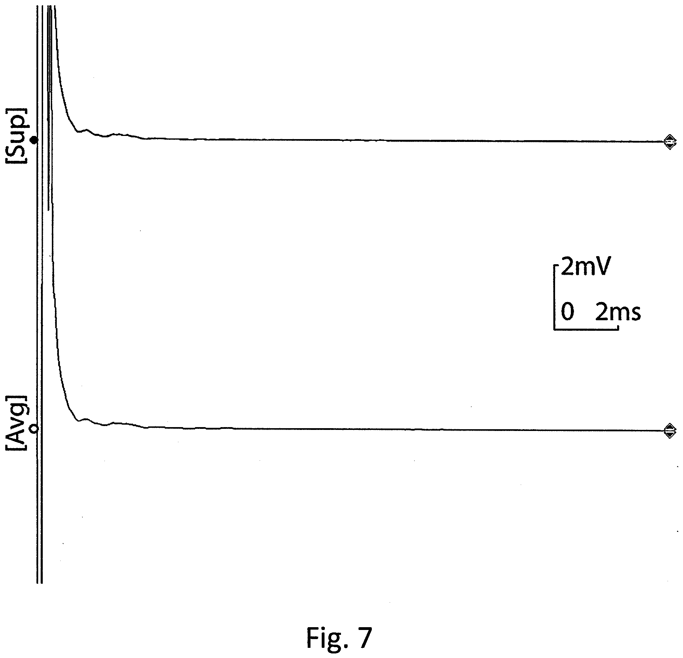

The most commonly used type of existing TMS compatible recording electrodes 1 is an electrode A on Fig. 4, and also an older type of TMS compatible recording electrodes 1 is an electrode B on Fig. 4, which is produced by the patent applicant but which is not the subject of this patent application, have been specifically selected as a reference representing the status of known recording electrodes 1. The results of both types are very similar. Fig. 6 and Fig. 7 show the waveform as recorded by the recording electrodes 1 of the type electrode A and electrode B, where the type B artifact is predominant in both cases as expected. In these cases, the type B artifact has an amplitude of the order of hundreds of µV and exponentially damped fading with a time constant of about 4 ms.

### Example 2

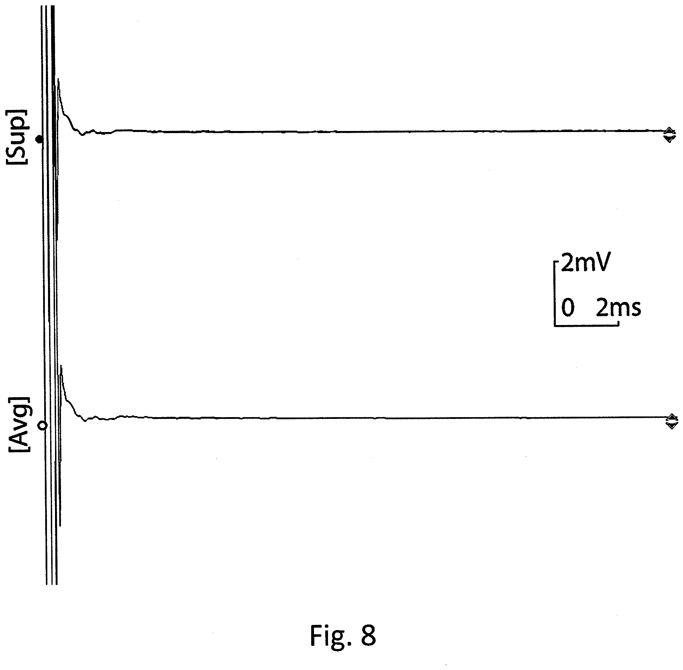

Next, a recording electrode 1 of a type electrode C was tested with three separate contact areas 3 with sizes of approx. 3 mm x 7 mm from Fig. 4, which were connected via resistors 4 of a resistance of 10 kΩ. Such high resistances of resistors 4 were chosen so that the formation of a relaxation artifact could not occur. The measured relaxation artifact was therefore created solely due to the size of these areas. Fig. 8 clearly shows the reduction and shortening of the relaxation artifact up to 2 ms. In contrast to the previous types of electrodes, however, an artifact caused by the heating of the recording electrode is present, which is manifested by the DC shift of the response curve as a result of the relatively large electrically separated contact areas 3.

### Example 3

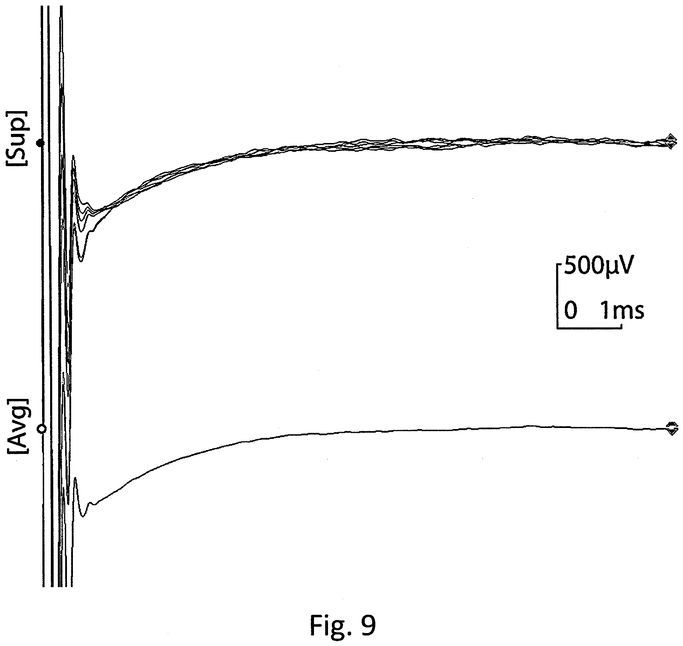

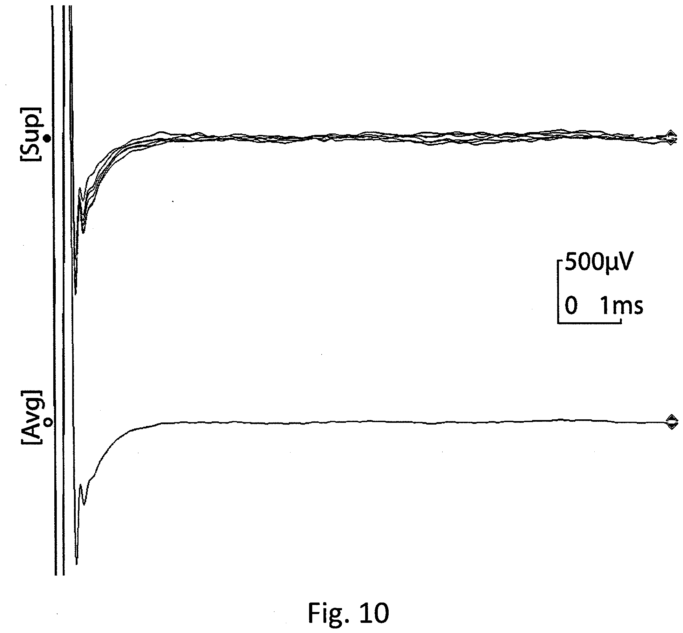

In order to better understand the benefit of the recording electrode 1 according to this invention, a comparison of two essentially identical recording electrodes 1 was made, one recording electrode 1 being the prior art electrode B from Fig. 4 and the other recording electrode 1 of the same type as electrode B from Fig. 4, but modified according to the present invention, i.e. it was divided into two separate contact areas 3 and each separate contact area 3 was connected to the output from the recording electrode 1 via a resistor 4 having a resistance of 100 Ω. Fig. 5 thus shows a prior art electrode E as recording electrode 1 connected to the output from the recording electrode 1 via resistor 4 of resistance of 50 Ω, and an electrode F from Fig. 4 as recording electrode 1, which was modified as described above according to the present invention by dividing the contact area 2 into two electrically separated contact areas 3, each of which is connected to the output from the recording electrode 1 via a 100 Ω resistor. Those skilled in the art will recognise that since both areas are connected in parallel to the output and measured biological tissue, the resulting resistances of the electrode E and electrode F as recording electrode 1 are also 50 Ω. The measurement results on both recording electrodes 1 are then shown in Fig. 9 and 10, where Fig. 9 shows the measurement result on the recording electrode 1 of type E from Fig. 5 and Fig. 10 the measurement result on the recording electrode 1 of type F from Fig. 5. By comparing the two measurement results, it is evident that type F recording electrode 1, i.e. according to the present invention, achieves an undistorted result after approx. 2 ms, while for the type E recording electrode 1 it was after up to approx. 5 ms. This is a significant difference.

### Example 4

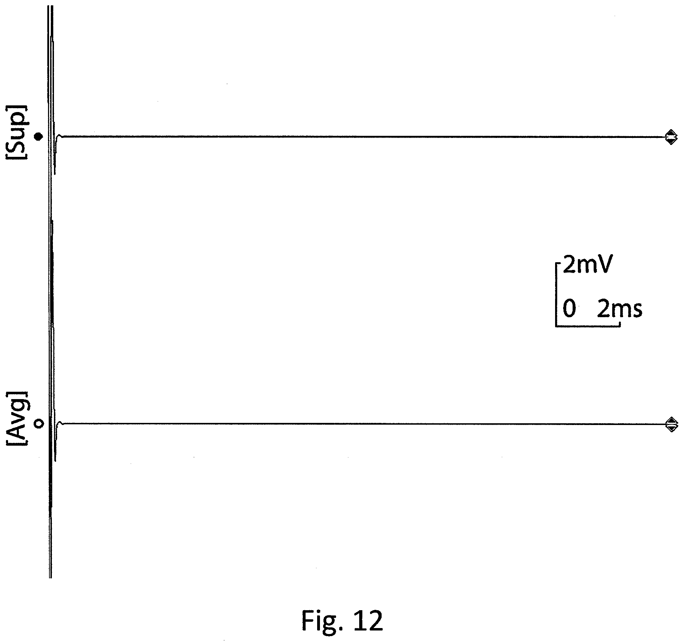

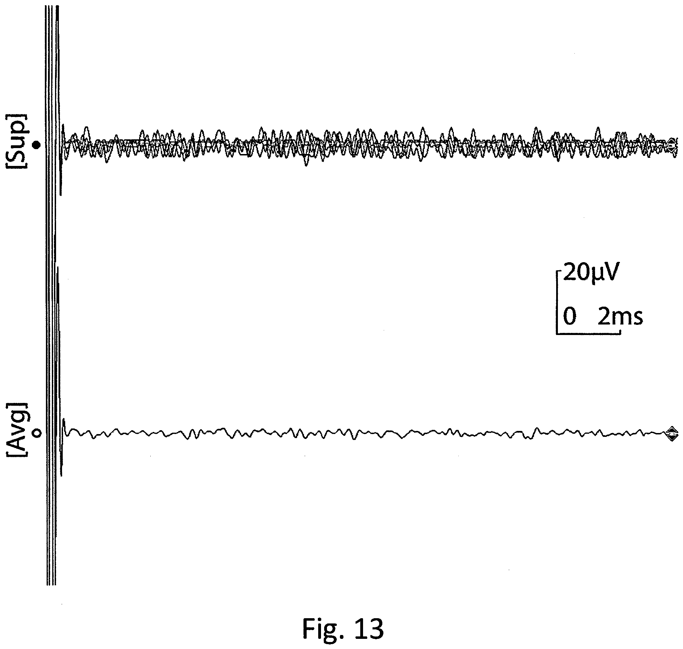

In addition, measurements were made on the type D recording electrode 1 from Fig. 4, which is provided with 12 separate contact areas 3 with dimensions of approx. 1.5 mm x 1.5 mm with separating resistors 4 with a size of 1,500 Ω each, which give the resulting resistance of 125 Ω on the output of the recording electrode 1. However, each two separate contact areas 3 are separated from each other by resistors 4 with a total resistance of 3,000 Ω. Fig. 12 and the 100x magnification in Fig. 13 clearly show that the undistorted result was achieved on the output in less than 1 ms.

### Example 5

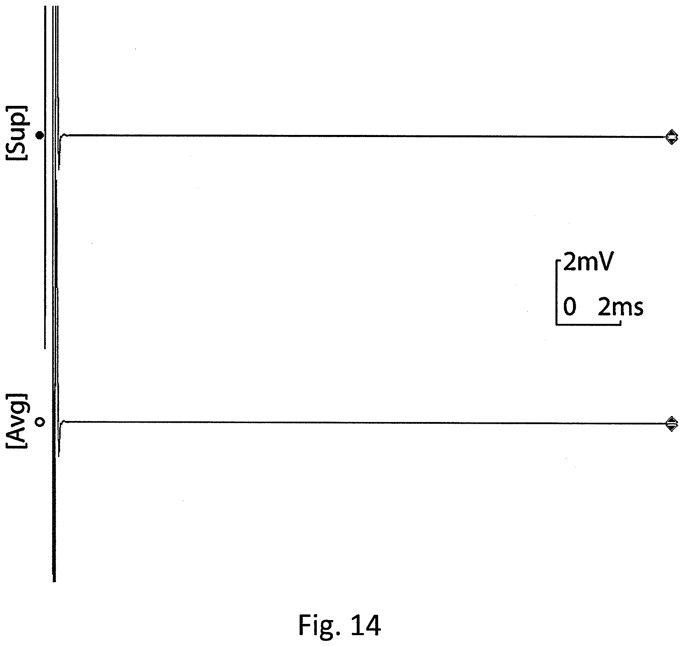

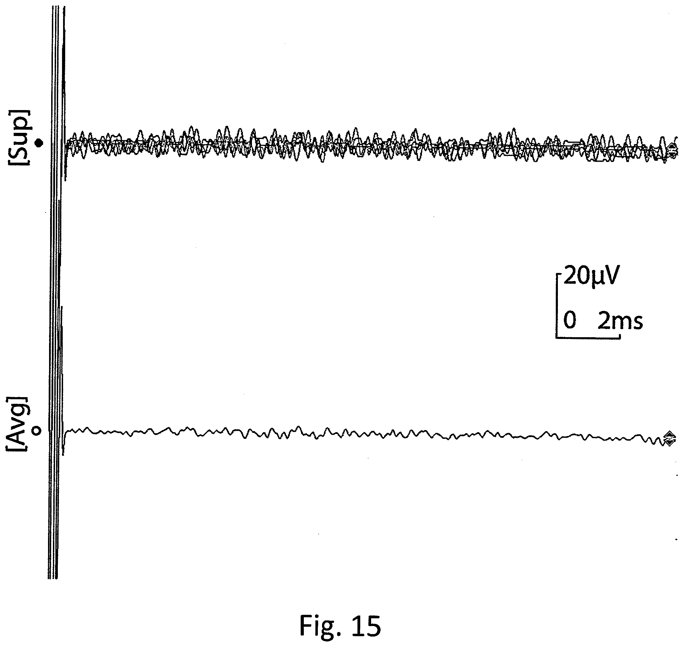

In addition, measurements were made on the type D recording electrode 1 from Fig. 4 with 12 separate contact areas 3 with dimensions of approx. 1.5 mm x 1.5 mm with separating resistors 4 with a size of 10 kΩ each, which give the resulting resistance of 833 Ω on the output of the recording electrode 1. Fig. 14 and the 100x magnification in Fig. 15 clearly show that the undistorted result was again achieved in less than 1 ms. When increasing the resistance size of resistors 4, there is no further improvement in artifact suppression.

It is clear to those skilled in the art that in order to be able to record an electrophysiological signal without the presence of relaxation artifacts, it is necessary to use 1 TMS compatible amplifier at the output from the recording electrode. The proposed TMS compatible recording electrode therefore describes a completely separate and independent problem and is not affected by the use or non-use of a TMS compatible amplifier.

It will be clear to those skilled in the art that the exemplary embodiments of the recording electrode, in particular as regards the exact size of the separate contact areas 3 of the recording electrode 1 and the number of these separate contact areas 3, as well as the used resistance sizes of resistors 4, are illustrative only and show particularly advantageous embodiments of the recording electrode 1 according to the present invention, if indicated in the description, and the subject matter of the invention is limited only by the appended patent claims.

### Industrial application

The proposed solution provides a new degree of TMS compatibility of the recording electrode, whereby an unspecified electrophysiological signal can be registered immediately after termination of the stimulation change of the magnetic induction over time under any orientation of the vector of the magnetic induction change (dB/dt vector) and spatial arrangement of the field lines. The prevention of formation of this effect cannot be solved by merely dividing the recording electrode into several parts, which are then electrically short-circuited to each other. Due to the construction of the recording electrode according to the invention, the generation of induced currents is reduced to the necessary minimum resulting from the physical nature of magnetic stimulation, without any additional interference from the recording electrode, thanks to which this recording electrode provides a quality record of electrophysiological activity just after TMS stimulation.

## Claims

1. Recording electrode (1) for a measurement of a electrophysiological signal from biological tissue, particularly suitable for use in magnetic stimulation, is provided with a contact area (2) designed to come into contact with a biological tissue by a direct contact or through an electro-conductive gel to facilitate an acquirement of the electrophysiological signal from the biological tissue to the contact area, where the contact area is electrically connected to an output of the recording electrode for conducting the electrophysiological signal for further processing, **characterised in that** the contact area (2) of the recording electrode (1) is divided into at least two separate contact areas (3) electrically separated from each other, each separate contact area (3) being connected to the output of the recording electrode (1) via an individual resistor (4), whose electrical resistance is at least 100 Ω, while the resulting resistance of all resistors connecting the individual separate contact areas (3) with the output of the recording electrode (1), as measured between the biological tissue and the output of the recording electrode (1), is at least 50 Ω, which corresponds to at least half the size of the impedance of the biological tissue in magnetic stimulation.

2. The recording electrode according to claim 1, **characterised in that** the resulting value of the electrical resistance of the resistors (4) through which the separate contact areas (3) are connected with the output from the recording electrode (1), is at least approx. 200 Ω.

3. The recording electrode according to claim 1 or 2, **characterised in that** the contact area (2) is divided into at least 3 separate contact areas (3), wherein the value of the electrical resistance of the resistor (4) through which each separate contact area (3) is connected with the output from the recording electrode (1), is at least approx. 1,000 Ω.

4. The recording electrode according to claim 1, **characterised in that** the contact area (2) is divided into at least 12 separate contact areas (3), wherein the value of the electrical resistance of the resistor (4) through which each separate contact area (3) is connected with the output from the recording electrode (1), is at least approx. 2,000 Ω.

5. The recording electrode according to claim 4, **characterised in that** the resulting value of the electrical resistance of the resistor (4) through which each separate contact area (3) is connected with the output from the recording electrode (1), is approx. 10,000 Ω.

6. The recording electrode according to any one of the preceding claims 1 to 5, **characterised in that** the size of each separate contact area (3) is approx. 1.5 mm x 1.5 mm.

## Patentansprüche

1. Aufzeichnungselektrode (1) für eine Messung eines elektrophysiologischen Signals von biologischem Gewebe, die insbesondere zur Verwendung bei Magnetstimulation geeignet ist und mit einer Kontaktfläche (2) versehen ist, die ausgelegt ist, mit einem biologischen Gewebe durch direkten Kontakt oder über ein elektrisch leitendes Gel in Kontakt zu kommen, um eine Erfassung des elektrophysiologischen Signals vom biologischen Gewebe zur Kontaktfläche zu ermöglichen, wobei die Kontaktfläche elektrisch mit einem Ausgang der Aufzeichnungselektrode zum Leiten des elektrophysiologischen Signals zum weiteren Verarbeiten verbunden ist, **dadurch gekennzeichnet, dass** die Kontaktfläche (2) der Aufzeichnungselektrode (1) in mindestens zwei separate Kontaktflächen (3) aufgeteilt ist, die elektrisch voneinander getrennt sind, wobei jede separate Kontaktfläche (3) mit dem Ausgang der Aufzeichnungselektrode (1) über ein individuelles Widerstandsbauelement (4) verbunden ist, dessen elektrischer Widerstand mindestens 100 Ω beträgt, während der resultierende Widerstand aller Widerstandsbauelemente, die die individuellen separaten Kontaktflächen (3) mit dem Ausgang der Aufzeichnungselektrode (1) verbinden, wie er zwischen dem biologischen Gewebe und dem Ausgang der Aufzeichnungselektrode (1) gemessen wird, mindestens 50 Ω beträgt, was mindestens einer Hälfte der Größe der Impedanz des biologischen Gewebes bei Magnetsimulation entspricht.

2. Aufzeichnungselektrode nach Anspruch 1, **dadurch gekennzeichnet, dass** der resultierende Wert des elektrischen Widerstands der Widerstandsbauelemente (4), über den die separaten Kontaktflächen (3) mit dem Ausgang der Aufzeichnungselektrode (1) verbunden sind, mindestens ungefähr 200 Ω beträgt.

3. Aufzeichnungselektrode nach Anspruch 1 oder 2, **dadurch gekennzeichnet, dass** die Kontaktfläche (2) in mindestens 3 separate Kontaktflächen (3) aufgeteilt ist, wobei der Wert des elektrischen Widerstands des Widerstandsbauelements (4), über den jede separate Kontaktfläche (3) mit dem Ausgang der Aufzeichnungselektrode (1) verbunden ist, mindestens ungefähr 1.000 Ω beträgt.

4. Aufzeichnungselektrode nach Anspruch 1, **dadurch gekennzeichnet, dass** die Kontaktfläche (2) in mindestens 12 separate Kontaktflächen (3) aufgeteilt ist, wobei der Wert des elektrischen Widerstands des Widerstandsbauelements (4), über den jede separate Kontaktfläche (3) mit dem Ausgang der Aufzeichnungselektrode (1) verbunden ist, mindestens ungefähr 2.000 Ω beträgt.

5. Aufzeichnungselektrode nach Anspruch 4, **dadurch gekennzeichnet, dass** der resultierende Wert des elektrischen Widerstands des Widerstandsbauelements (4), über den jede separate Kontaktfläche (3) mit dem Ausgang der Aufzeichnungselektrode (1) verbunden sind, ungefähr 10.000 Ω beträgt.

6. Aufzeichnungselektrode nach einem der vorangehenden Ansprüche 1 bis 5, **dadurch gekennzeichnet, dass** die Größe jeder separaten Kontaktfläche (3) ungefähr 1,5 mm x 1,5 mm beträgt.

## Revendications

1. Électrode d'enregistrement (1) pour une mesure d'un signal électrophysiologique provenant d'un tissu biologique, en particulier appropriée pour une utilisation en stimulation magnétique, qui comporte une zone de contact (2) conçue pour entrer en contact avec un tissu biologique par un contact direct ou par l'intermédiaire d'un gel électroconducteur pour faciliter une acquisition du signal électrophysiologique provenant du tissu biologique sur la zone de contact, la zone de contact étant connectée électriquement à une sortie de l'électrode d'enregistrement pour conduire le signal électrophysiologique en vue d'un traitement ultérieur, **caractérisée par le fait que** la zone de contact (2) de l'électrode d'enregistrement (1) est divisée en au moins deux zones de contact distinctes (3) électriquement séparées les unes des autres, chaque zone de contact distincte (3) étant connectée à la sortie de l'électrode d'enregistrement (1) par l'intermédiaire d'une résistance individuelle (4), dont la résistance électrique est d'au moins 100 Ω, tandis que la résistance résultante de toutes les résistances connectant les zones de contact distinctes (3) individuelles à la sortie de l'électrode d'enregistrement (1), telle que mesurée entre le tissu biologique et la sortie de l'électrode d'enregistrement (1), est d'au moins 50 Ω, ce qui correspond à au moins la moitié de la taille de l'impédance du tissu biologique en stimulation magnétique.

2. Électrode d'enregistrement selon la revendication 1, **caractérisée par le fait que** la valeur résultante de la résistance électrique des résistances (4), par lesquelles les zones de contact distinctes (3) sont connectées à la sortie de l'électrode d'enregistrement (1), est au moins d'approximativement 200 Ω.

3. Électrode d'enregistrement selon la revendication 1 ou 2, **caractérisée par le fait que** la zone de contact (2) est divisée en au moins 3 zones de contact distinctes (3), la valeur de la résistance électrique de la résistance (4) par laquelle chaque zone de contact distincte (3) est connectée à la sortie de l'électrode d'enregistrement (1) étant au moins d'approximativement 1000 Ω.

4. Électrode d'enregistrement selon la revendication 1, **caractérisée par le fait que** la zone de contact (2) est divisée en au moins 12 zones de contact distinctes (3), la valeur de la résistance électrique de la résistance (4) par laquelle chaque zone de contact distincte (3) est connectée à la sortie de l'électrode d'enregistrement (1) étant au moins d'approximativement 2000 Ω.

5. Électrode d'enregistrement selon la revendication 4, **caractérisée par le fait que** la valeur résultante de la résistance électrique de la résistance (4), par laquelle chaque zone de contact distincte (3) est connectée à la sortie de l'électrode d'enregistrement (1), est d'approximativement 10 000 Ω.

6. Électrode d'enregistrement selon l'une quelconque des revendications précédentes 1 à 5, **caractérisée par le fait que** la taille de chaque zone de contact distincte (3) est d'approximativement 1,5 mm x 1,5 mm.
